# FASCICULE DE BREVET EUROPEEN

(11) **EP 0 475 851 B1**
(45) Date de publication et mention de la délivrance du brevet: **22.11.1995**
(21) Numéro de dépôt: 91402442.7
(22) Date de dépôt: 13.09.1991
(51) Int. Cl.: C09K 15/04, C11B 5/00, A61K 7/48

(54) **Système anti-oxydant à base d'un acide aminé basique et d'un tocophérol**
Antioxydantsystem auf Basis einer basischen Aminosäure und eines Tocopherols
Antioxydant system based on basic aminoacid and a tocopherol

(30) Priorité: 14.09.1990 FR 9011384
(43) Date de publication de la demande: 18.03.1992
(73) Titulaire: L'OREAL, 75008 Paris (FR)
(72) Inventeur: N'Guyen, Quang Lan, F-92160 Antony (FR); Millecamps, François, F-94230 Cachan (FR); Galey, Jean-Baptiste, F-75005 Paris (FR)
(74) Mandataire: Stalla-Bourdillon, Bernard

(56) Documents cités:
- EP-A- 0 280 606
- EP-A- 0 353 161
- GB-A- 1 193 027
- PATENT OFFICE OF JAPAN, FILE SUPPLIER JAPS, accession no. 80-39141C [22], Tokyo, JP; & JP-A-55 053 210.
- WORLD PATENT INDEX, accession no. 78-11506A [06], Derwent Publications Ltd, Londres, GB; & JP-A-52 156 787.
- WORLD PATENT INDEX, LATEST, accession no. 89-057364 [08], Derwent Publications Ltd, Londres, GB; & JP-A-1 009 285.

## Description

La présente invention a pour objet un nouveau système anti-oxydant à base d'un acide aminé basique en association avec au moins un tocophérol ou au moins un de ses dérivés et au moins un polypeptide non thiolé ledit système étant exempt d'agent complexant, l'utilisation d'un tel système anti-oxydant et les compositions à base de matières oléagineuses contenant un tel système, notamment des compositions cosmétiques.

On sait que les corps gras ont tendance à s'oxyder, même à température ambiante, et cette oxydation (ou rancissement) leur fait acquérir de nouvelles propriétés, notamment gustatives ou olfactives qui sont généralement considérées comme indésirables lorsque ces corps gras sont incorporés, par exemple, dans des compositions alimentaires ou dans des compositions cosmétiques.

On utilise donc couramment, dans les compositions contenant des corps gras, des agents protecteurs qui jouent en fait le rôle d'anti-oxydant.

Parmi les anti-oxydants connus, on utilise couramment l'acide ascorbique qui agit notamment par absorption directe d'oxygène. Toutefois, l'acide ascorbique est très peu soluble dans les corps gras et est donc difficilement utilisable pour protéger ceux-ci contre l'oxydation.

Afin de solubiliser la molécule d'acide ascorbique dans les matières grasses, on a proposé l'utilisation de divers esters d'ascorbyle tels que par exemple le stéarate, le palmitate ou le laurate d'ascorbyle; voir par exemple l'article de C.F. BOURGEOIS, "Revue Française des Corps Gras", n°9, pages 353-356 (Septembre 1981).

On sait qu'en dehors de leurs propriétés anti-oxydantes propres, les dérivés ascorbiques ont également la propriété d'améliorer l'activité d'agents anti-oxydants tels que les tocophérols ou l'acide caféique et ses esters, en favorisant la régénération de ces agents anti-oxydants; voir par exemple H.S. OLCOTT, "Oil Soap", 18, (1941), 77, le brevet US 2.462.663 ainsi que la demande de brevet français n° 75.25621 (2.282.266).

On a également proposé diverses améliorations de ces agents anti-oxydants binaires, du type dérivés ascorbiques + tocophérols ou dérivés ascorbiques + dérivés caféiques, en prévoyant l'addition d'un troisième constituant améliorant encore les effets anti-oxydants. Parmi les troisièmes constituants de ces systèmes ternaires, on peut citer notamment l'acide p-aminobenzoïque (brevet US 2.462.663). des phospholipides (R.W. RIEMENSCHNEIDER et al., "Oil Soap" (1944), 47), des amines (KLAUI, "The Functional (Technical) Uses of Vitamins", ed. by M. STEIN, University of Nottingham Seminar Vitamins, London, England (1971), page 110), etc...

Il a par ailleurs été décrit dans la demande de brevet français n° 88.10295 qu'il est possible d'améliorer notablement les propriétés anti-oxydantes des esters d'ascorbyle en utilisant ces anti-oxydants conjointement avec au moins un tocophérol ou un mélange de tocophérols ou de l'acide caféique ou l'un de ses dérivés, au moins un agent complexant et au moins un polypeptide non thiolé. Ce système présente cependant des inconvénients inhérents à la présence des esters d'ascorbyle. En effet ceux-ci, dans certaines conditions, provoquent un effet de jaunissement des compositions.

Il a en outre été décrit dans GB 1.193.027 que l'addition à un agent anti-oxydant tel qu'un tocophérol, de composés tels que des acides aminés et notamment basiques ou d'acide ascorbique ou l'un de ses esters d'acide gras permettait l'obtention d'un effet de synergie.

On a maintenant découvert qu'il est possible d'éviter ou de réduire les inconvénients de l'état de la technique et d'obtenir à la fois une potentialisation importante de l'effet anti-oxydant, en utilisant un acide aminé basique en association avec au moins un tocophérol ou un dérivé de tocophérol et au moins un polypeptide non thiolé ladite association étant exempte d'agent complexant.

Certains acides aminés ont été décrits comme anti-oxydants par T. RIISOM et coll. (J.A.O.C.S. Octobre 1980, pages 354-358).

D'autre part, A. SEHER et coll. ont étudié l'effet anti-oxydant d'un mélange d'acides aminés extraits de plantes (Fette Seifen Anstrichmittel, Vol.88, n°1, 1986, pages 1-42) et ont constaté que cet effet est augmenté par addition d'α-tocophérol notamment.

Par ailleurs, il est décrit dans JP 1.009.285 un système anti-oxydant à base d'acides aminés basiques pouvant être éventuellement associé à divers agents anti-oxydants tels qu'un tocophérol ou de l'acide ascorbique.

La présente invention a donc pour objet un nouveau système anti-oxydant à base d'au moins un acide aminé basique caractérisé par le fait qu'il comprend au moins un tocophérol ou un dérivé de tocophérol et au moins un polypeptide non thiolé ladite association étant exempte d'agent complexant.

Par acide aminé basique on entend les acides aminés basiques naturels tels que par exemple la lysine, l'arginine et l'histidine dans toutes leurs formes isomériques ou racémiques ainsi que les acides aminés basiques synthétiques et les dérivés des acides aminés naturels. De préférence, on utilise selon l'invention la lysine ou l'arginine.

Par l'expression "tocophérol", on entend non seulement l'α tocophérol mais également le β, γ ou δ tocophérol ainsi que leurs mélanges. Parmi les dérivés de tocophérols, or peut citer les esters de tocophérol tels que l'acétate de tocophérol et le nicotinate de tocophérol.

Le polypeptide non thiolé du système anti-oxydant selon l'invention a, d'une manière générale, un poids moléculaire moyen compris entre environ 1.000 et environ 100.000. Parmi les polypeptides que l'on peut utiliser, on peut en particulier mentionner les suivants:
(a) le polypeptide vendu sous la dénomination de "KERASOL®" (polypeptide de la kératine soluble de poids moléculaire moyen d'environ 100.000) par la Société CRODA Chemicals Ltd,
(b) le polypeptide vendu sous la dénomination de "Polypeptide SF®" (polypeptide de collagène animal partiellement neutralisé de poids moléculaire moyen d'environ 1.000) par la Société NAARDEN,
(c) le polypeptide vendu sous la dénomination de "Polypeptide LSN®" (polypeptide de collagène animal sous forme de sel d'ammonium contenant environ 3% au maximum de sel inorganique) par la Société NAARDEN, et
(d) le polypeptide vendu sous la dénomination de "LACTOLAN®" (polypeptide obtenu à partir du lait frais de vache préalablement délipidé) par les LABORATOIRES SEROBIOLOGIQUES de NANCY.

On a constaté de façon tout-à-fait surprenante que les résultats de l'activité anti-oxydante du système selon l'invention montre un effet de synergie important par rapport aux composés pris séparément ainsi que par rapport aux associations binaires.

Selon l'invention, le système anti-oxydant est de préférence constitué de:
0,5 à 20% de tocophérol(s) ou de dérivé(s) de tocophérol(s)
0,5 à 50% d'acide(s) aminé(s) basique(s) et
0,5 à 90%, de polypeptide(s) non thiolé(s)
Le rapport préféré entre la concentration d'acide aminé basique et la concentration de tocophérol(s) est compris entre 1 et 20.

L'efficacité du système anti-oxydant selon l'invention a été démontrée par la méthode d'oxydation accélérée de la vitamine F, qui est une substance particulièrement sensible à l'oxydation.

Pour l'étude, on utilise le dispositif automatique "Rancimat®" de la Société Metrohm.

On prépare des mélanges dans la vitamine F avec différentes quantités d'un tocophérol seul, d'un acide aminé basique seul et d'un polypeptide non thiolé seul ainsi qu'un des systèmes binaires d'un tocophérol et d'un acide aminé basique, d'un tocophérol et d'un polypeptide non thiolé, ou encore d'un acide aminé basique et d'un polypeptide non thiolé. Ces mélanges sont comparés au système ternaire selon l'invention ainsi qu'à un témoin.

On porte chaque échantillon à 100°C, sous un barbotage d'air (20 litres/heure). On suit alors en continu la concentration en acides volatils résultant de la dégradation des hydroperoxydes et des aldéhydes de vitamine F, dans une cellule remplie d'eau dans laquelle on plonge une électrode en platine. Cette électrode mesure, en fonction du temps, l'augmentation de la conductivité provoquée par l'augmentation de la concentration d'acides volatils. Le temps d'induction sera déterminé par l'intersection des deux asymptotes de la courbe d'oxydation exponentielle obtenue.

Ce temps correspond au temps de latence précédent l'autoxydation de la vitamine F. Plus ce temps de latence est long, meilleure est la résistance de la vitamine F à l'autoxydation.

Les résultats obtenus sont rassemblés dans le tableau suivant :

| TOCOPHEROL | ACIDE AMINE BASIQUE (Lysine) | POLYPEPTIDE NON THIOLE (Lactolan) | TEMPS D' INDUCTlON en mn |
|---|---|---|---|
| - | - | - | 18 |
| 0,1% | - | - | 80 |
| - | 0,5% | - | 125 |
| - | - | 5% | 35 |
| 0,1% | 0,5% | - | 480 |
| - | 0,5% | 5% | 72 |
| 0,1% | - | 5% | 220 |
| 0,1% | 0,5% | 5% | 1100 |

Ces résultats montrent nettement la supériorité de l'activité anti-oxydante du système ternaire selon l'invention par rapport aux constituants pris séparément, et par rapport aux systèmes binaires tocophérol-lysine, tocophérol-polypeptide non thiolé et lysine-polypeptide non-thiolé.

L'invention a également pour objet des compositions contenant des corps gras, caractérisées par le fait qu'elles renferment au moins un système anti-oxydant tel que défini précédemment.

Les compositions de l'invention peuvent être notamment des compositions alimentaires (huiles comestibles, saindoux, beurre, margarine ou autres succédanés de beurre), des compositions cosmétiques ou dermo-pharmaceutiques.

Les corps gras présents dans les compositions de l'invention sont par exemple des corps gras d'origine animale tels que la cétine (blanc de baleine), la cire d'abeille, la lanoline, le perhydrosqualène, l'huile de tortue, etc...; des corps gras végétaux sous forme d'huiles, de graisses ou de cires tels que l'huile d'amande douce, l'huile d'avocat, l'huile d'olive,...; les huiles de coprah ou de palmiste hydrogénées, le beurre de cacao, la cire de Carnauba, la cire de Montana ; ainsi que des huiles synthétiques constituées par des esters et/ou éthers de glycérol ou de glycols tels que par exemple ceux qui sont décrits dans les brevets français n°75.24656, 75.24657 et 75.24658.

En plus des corps gras plus ou moins oxydables, les compositions cosmétiques ou dermo-pharmaceutiques peuvent contenir des produits sensibles à l'oxydation tels que par exemple de la vitamine F ou du β-carotène.

Les compositions selon l'invention se présentent sous la forme de solutions huileuses, d'émulsions E/H ou H/E, de produits solides éventuellement anhydres, de lotions ou encore de micro-dispersions, de vésicules lipidiques ioniques ou non ioniques. Elles constituent notamment des laits pour les soins de la peau, des crèmes (crèmes pour le visage, pour les mains, pour le corps, crèmes anti-solaires, crèmes démaquillantes, crèmes fonds de teint), des fonds de teint fluides, des laits démaquillants, des laits anti-solaires, des huiles pour le bain, des rouges à lèvres, des fards à paupières, des sticks déodorants, etc...

Pour l'application par voie topique, les compositions pharmaceutiques selon l'invention comprennent les véhicules et ingrédients nécessaires pour permettre de présenter la composition par exemple sous la forme d'onguents, de crèmes, de laits, de pommades et de solutions huileuses.

Selon une forme de réalisation préférée, les compositions cosmétiques ou dermo-pharmaceutiques se présentent sous une forme destinée à être appliquée par voie topique, en particulier de crèmes destinées à la protection de l'oxydation des lipides de la peau.

Dans les compositions selon l'invention, le système anti-oxydant tel que défini ci-dessus est généralement présent de sorte que l'on ait les proportions suivantes par rapport au poids total de la composition:

| | |
|---|---|
| - Tocophérol(s) ou dérivé(s) | 0,05 à 2% |
| - Acide aminé basique | 0,05 à 5% |
| - Polypeptide non thiolé (en matière active) | 0,05 à 8% |

Les compositions de l'invention peuvent en outre contenir des composés actifs ou des ingrédients utilisés de façon usuelle dans les compositions mentionnées ci-dessus, tels que des agents tensio-actifs, des colorants, des parfums, des produits astringents, des produits absorbant l'ultra-violet, des solvants organiques, de l'eau, etc...

Ces compositions sont préparées selon les méthodes usuelles.

On va maintenant donner à titre d'illustration plusieurs exemples de systèmes anti-oxydants selon l'invention ainsi que des exemples de compositions contenant de tels systèmes anti-oxydants.

### EXEMPLE 1

| | |
|---|---|
| - Tocophérols (mélange α, β, etδ) | 2,45% |
| - Lysine | 16,25% |
| - Polypeptide "KERASOL" (en matière active) | 81,30% |

### EXEMPLE 2

| | |
|---|---|
| - Tocophérols | 20% |
| - Arginine | 40% |
| - Polypeptide "LACTOLAN" (en matière active) | 40% |

### EXEMPLE 3

| | |
|---|---|
| - Tocophérols | 8,80% |
| - Lysine | 3,50% |
| - Polypeptide "SF" (en matière active) | 87,70% |

### EXEMPLES DE COMPOSITIONS COSMETIQUES OU DERMO-PHARMACEUTIQUES

### A. Crème anti-inflammatoire pour le traitement de l'eczéma

| | |
|---|---|
| - Lanolate de magnésium | 14,4% |
| - Alcool de lanoline | 3,6% |
| - Huile de tournesol | 40% |
| - Myristate d'isopropyle | 8% |
| - Ozokérite | 4% |
| - Vitamine F | 2% |
| - 17-propionate 21-acétate d'hydrocotisone | 0,1% |
| - Lécithine de soja | 5% |
| - Tocophérols | 0,15% |
| - Lysine | 1% |
| - Polypeptide "KERASOL" | 5% |
| - Parfum | 0,8% |
| - Parahydroxybenzoate de méthyle | 0,3% |
| - Eau qsp | 100% en poids |

### B. Huile corporelle

| | |
|---|---|
| - Huile de karité | 2% |
| - Huile de tournesol | 31,8% |
| - Vitamine F | 2% |
| - Huile de soja | 32% |
| - Tocophérols | 1% |
| - Arginine | 2% |
| - Polypeptide "LACTOLAN" | 2% |
| - Lécithine de soja | 0,10% |
| - Huile d'arachide qsp | 100% en poids |

### C. Fluide de soin pour le corps

### 1ère phase

Lipide amphiphile non ionique de formule générale 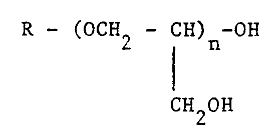
dans laquelle R est un radical hexadécyle
et n a une valeur statistique

| | |
|---|---|
| moyenne égale à 3 | 4,5% |
| Cholesterol | 4,5% |
| Dicétylphosphate | 1% |
| Parahydroxybenzoate de méthyle | 0,3% |
| Eau déminéralisée stérile | 30% |

On agite vigoureusement ce mélange afin d'obtenir une dispersion homogène de sphérules.

### 2ème phase

On ajoute à la dispersion de sphérules obtenue dans la première phase les substances suivantes :

| | |
|---|---|
| Parfum | 0,4% |
| Huile de tournesol | 10% |
| Huile de paraffine | 4% |
| Vitamine F | 2% |
| Tocophérols | 0,15% |
| Lysine | 1% |
| Polypeptide "SF" | 3% |
| Polymère carboxyvinylique vendu sous la dénomination de "Carbopol 940" par la Société Goodrich | 0,4% |
| Triéthanolamine | 0,4% |
| Eau déminéralisée qsp | 100% |

### D. Crème liposomée

### 1ère phase

| | |
|---|---|
| Lécithine hydrogénée de soja vendue sous la dénomination "Lecinol 510" par la Société NIKKO | 1,8% |
| Cholestérol | 0,9% |
| Lipoamino-acide palmitoyl collagénique de formule CH₃-(CH₂)₁₄-CO-NH-CHR-COOH dans laquelle R est un reste d'acides aminés obtenu par hydrolyse du collagène, vendu sous la dénomination "PCO" par la Société RHONE POULENC | 0,3% |
| Parahydroxybenzoate de méthyle | 0,25% |
| Eau déminéralisée stérile | 30% |

On agite vigoureusement ce mélange afin d'obtenir une dispersion homogène de sphérules

### 2ème phase

On ajoute à la dispersion de sphérules de la première phase les substances suivantes :

| | |
|---|---|
| Parfum | 0,4% |
| Huile de karité | 10% |
| Huile d'amande | 7% |
| Cyclométhicone | 5% |
| Tocophérols | 0,15% |
| Lysine | 1% |
| Polypeptide "LACTOLAN" | 0,5% |
| Polymère carboxyvinylique vendu sous la dénomination "CARBOPOL 940" par la Société GOODRICH | 0,4% |
| Triéthanolamine | 0,4% |
| Eau déminéralisée stérile qsp | 100% |

### E. Lait corporel huile-dans-l'eau

| | |
|---|---|
| Stéarate de glycérol | 2% |
| Monostéarate de sorbitan à 20 moles d'oxyde d'éthylène vendu sous la dénomination "TWEEN 60" par la Société ATLAS | 1% |
| Acide stéarique | 1,4% |
| Triéthanolamine | 0,7% |
| Carbopol 940 neutralisé par de la triéthanolamine | 0,2% |
| Huile d'amande douce | 3% |
| Huile de vaseline | 8% |
| Tocophérols | 0,1% |
| Lysine | 1% |
| Polypeptide "LACTOLAN" | 3% |
| Conservateurs qs | |
| Eau déminéralisée stérile qsp | 100% |

### F. Crème de soin huile-dans-l'eau

| | |
|---|---|
| Stéarate de glycérol | 2% |
| Tween 60 | 1% |
| Alcool cétylique | 0,5% |
| Acide stéarique | 1,4% |
| Triéthanolamine | 0,7% |
| Carbopol 940 neutralisé par de la triéthanolamine | 0,4% |
| Fraction liquide de graisse de karité | 12% |
| Perhydrosqualène de synthèse | 12% |
| Tocophérols | 0,2% |
| Arginine | 2% |
| Polypeptide "SF" | 3% |
| Conservateurs qs | |
| Eau déminéralisée stérile qsp | 100% |

## Revendications

1. Système anti-oxydant à base d'au moins un acide aminé basique, caractérisé par le fait qu'il comprend au moins un tocophérol ou un dérivé de tocophérol et au moins un polypeptide non thiolé, ledite système étant exempt d'agent complexant.

2. Système anti-oxydant selon la revendication 1, caractérisé par le fait que l'acide aminé basique est l'arginine ou la lysine ou leurs mélanges.

3. Système anti-oxydant selon la revendication 1 ou 2, caractérisé par le fait que le tocophérol est choisi parmi l'α-tocophérol, le β-tocophérol, le γ-tocophérol, le δ-tocophérol, et leurs mélanges.

4. Système anti-oxydant selon la revendication 1 ou 2, caractérisé par le fait que le dérivé de tocophérol est choisi parmi l'acétate de tocophérol et le nicotinate de tocophérol.

5. Système anti-oxydant selon la revendication 1 ou 2, caractérise par le fait que le polypeptide non thiolé a un poids moléculaire compris entre 1.000 et 100.000.

6. Système anti-oxydant selon l'une quelconque des revendications précédentes caractérisé par le fait que l'acide aminé basique est présent à raison de 0,5 à 50% en poids.

7. Système anti-oxydant selon l'une quelconque des revendications précédentes, caractérisé par le fait que le polypeptide non thiolé est présent à raison de 0,5 à 90% en poids.

8. Système anti-oxydant selon l'une quelconque des revendications précédentes, caractérisé par le fait que le tocophérol ou le dérivé de tocophérol est présent à raison de 0,5 à 20 % en poids.

9. Système anti-oxydant selon l'une quelconque des revendications précédentes, caractérisé par le fait que le rapport entre la concentration d'acide aminé basique et la concentration de tocophérol ou du dérivé de tocophérol est compris entre 1 et 20.

10. Système anti-oxydant selon l'une quelconque des revendications précédentes, caractérisé par le fait qu'il est constitué de:
0,5 à 20% de tocophérol(s) ou de dérivé(s) de tocophérol(s)
0,5 à 50% d'acide(s) aminé(s) basique(s) et
0,5 à 90% de polypeptide(s) non thiolé(s).

11. Composition contenant des corps gras, caractérisée par le fait qu'elle contient un système anti-oxydant selon l'une quelconque des revendications 1 à 10.

12. Composition cosmétique ou pharmaceutique, caractérisée par le fait qu'elle contient un système anti-oxydant selon l'une quelconque des revendications 1 à 10, les proportions des constituants du système anti-oxydant par rapport au poids total de la composition étant de:
| | |
|---|---|
| - Tocophérol(s) ou dérivé(s) de tocophérol(s) | 0,05 à 2% |
| - Acide(s) aminé(s) basique(s) | 0,05 à 5% |
| - Polypeptide non thiolé (en matière active) | 0,05 à 8% |

13. Composition selon la revendication 12, caractérisée par le fait que l'acide aminé basique est la lysine ou l'arginine.

14. Composition selon l'une quelconque des revendications 12 et 13, caractérisée par le fait que le tocophérol est choisi parmi l' α-tocophérol, le β-tocophérol, le γ-tocophérol, le δ-tocophérol et leurs mélanges.

15. Composition selon l'une quelconque des revendications 12 et 13, caractérisée par le fait que le dérivé de tocophérol est choisi parmi l'acétate de tocophérol et le nicotinate de tocophérol.

16. Composition selon l'une quelconque des revendications 12 à 15, caractérisée par le fait qu'elle se présente sous forme d'une crème destinée à la protection de l'oxydation des lipides de la peau.

## Claims

1. Antioxidant system based on at least one basic amino acid, characterized in that it comprises at least one tocopherol or one tocopherol derivative and at least one unthiolated polypeptide, the said system being free of complexing agent.

2. Antioxidant system according to Claim 1, characterized in that the basic amino acid is arginine or lysine or mixtures thereof.

3. Antioxidant system according to Claim 1 or 2, characterized in that the tocopherol is chosen from α-tocopherol, β-tocopherol, γ-tocopherol, δ-tocopherol and mixtures thereof.

4. Antioxidant system according to Claim 1 or 2, characterized in that the tocopherol derivative is chosen from tocopheryl acetate and tocopheryl nicotinate.

5. Antioxidant system according to Claim 1 or 2, characterized in that the unthiolated polypeptide has a molecular weight between 1,000 and 100,000.

6. Antioxidant system according to any one of the preceding claims, characterized in that the basic amino acid is present to an amount of 0.5 to 50% by weight.

7. Antioxidant system according to any one of the preceding claims, characterized in that the unthiolated polypeptide is present to an amount of 0.5 to 90% by weight.

8. Antioxidant system according to any one of the preceding claims, characterized in that the tocopherol or the tocopherol derivative is present to an amount of 0.5 to 20% by weight.

9. Antioxidant system according to any one of the preceding claims, characterized in that the ratio of the concentration of basic amino acid to the concentration of tocopherol or of the tocopherol derivative is between 1 and 20.

10. Antioxidant system according to any one of the preceding claims, characterized in that it consists of:
0.5 to 20% of tocopherol(s) or of tocopherol derivative(s)
0.5 to 50% of basic amino acid(s) and
0.5 to 90% of unthiolated polypeptide(s).

11. Composition containing fatty substances, characterized in that it contains an antioxidant system according to any one of Claims 1 to 10.

12. Cosmetic or pharmaceutical composition, characterized in that it contains an antioxidant system according to any one of Claims 1 to 10, the proportions of the constituents of the antioxidant system relative to the total weight of the composition being:
| | |
|---|---|
| - tocopherol(s) or tocopherol derivative(s) | 0.05 to 2% |
| - basic amino acid(s) | 0.05 to 5% |
| - unthiolated polypeptide (as active material) | 0.05 to 8% |

13. Composition according to Claim 12, characterized in that the basic amino acid is lysine or arginine.

14. Composition according to either of Claims 12 and 13, characterized in that the tocopherol is chosen from α-tocopherol, β-tocopherol, γ-tocopherolX δ-tocopherol and mixtures thereof.

15. Composition according to either of Claims 12 and 13, characterized in that the tocopherol derivative is chosen from tocopheryl acetate and tocopheryl nicotinate.

16. Composition according to any one of Claims 12 to 15, characterized in that it is in the form of a cream intended to protect the lipids of the skin against oxidation.

## Patentansprüche

1. Antioxidationssystem auf der Grundlage mindestens einer basischen Aminosäure, dadurch gekennzeichnet, daß es mindestens ein Tocopherol oder ein Derivat des Tocopherols sowie mindestens ein nichtthioliertes Polypeptid enthält, wobei das System frei von Komplexiermitteln ist.

2. Antioxidationssystem nach Anspruch 1 , dadurch gekennzeichnet, daß die basische Aminosäure Arginin oder Lysin bzw. deren Mischungen ist.

3. Antioxidationssystem nach Anspruch 1 oder 2, dadurch gekennzeichnet, daß das Tocopherol aus 1'-α-Tocopherol, β-Tocopherol, γ-Tocopherol, δ-Tocopherol sowie deren Gemischen ausgewählt ist.

4. Antioxidationssystem nach Anspruch 1 oder 2, dadurch gekennzeichnet, daß das Tocopherolderivat aus Tocopherolacetat und Tocopherolnikotinat ausgewählt ist.

5. Antioxidationssystem nach Anspruch 1 oder 2, dadurch gekennzeichnet, daß das nichtthiolierte Polypeptid ein Molekulargewicht im Bereich zwischen 1.000 und 100.000 aufweist.

6. Antioxidationssystem nach einem der vorstehenden Ansprüche, dadurch gekennzeichnet, daß die basische Aminosäure in einer Menge von 0,5 bis 50 Gew.% vorhanden ist.

7. Antioxidationssystem nach einem der vorstehenden Ansprüche, dadurch gekennzeichnet, daß das nichtthiolierte Polypeptid in einer Menge von 0,5 bis 90 Gew.% vorhanden ist.

8. Antioxidationssystem nach einem der vorstehenden Ansprüche, dadurch gekennzeichnet, daß das Tocopherol oder das Tocopherolderivat in einer Menge von 0,5 bis 20 Gew.% vorhanden ist.

9. Antioxidationssystem nach einem der vorstehenden Ansprüche, dadurch gekennzeichnet, daß das Konzentrationsverhältnis von basischer Aminosäure zu Tocopherol bzw. einem Derivat davon zwischen 1 und 20 beträgt.

10. Antioxidationssystem nach einem der vorstehenden Ansprüche, dadurch gekennzeichnet, daß es aus den folgenden Komponenten besteht:
0,5 bis 20 % ein oder mehrere Tocopherole bzw. seinen Derivaten,
0,5 bis 50 % eine oder mehrere basische Aminosäuren, sowie
0,5 bis 90 % ein oder mehrere nichtthiolierte Polypeptide.

11. Zusammensetzung mit einem Gehalt an Fettstoffen, dadurch gekennzeichnet, daß sie ein Antioxidationssystem nach einem der Ansprüche 1 bis 10 enthält.

12. Kosmetische Zubereitung oder Arzneimittel, dadurch gekennzeichnet, daß sie jeweils ein Antioxidationssystem nach einem der Ansprüche 1 bis 10 enthalten, wobei die Mengenverhältnisse der Bestandteile des Antioxidationssystems in bezug auf das Gesamtgewicht der Zusammensetzung wie folgt betragen:
| | |
|---|---|
| - ein oder mehrere Tocopherole bzw. dessen Derivate | 0,05 bis 2 % |
| - eine oder mehrere basische Aminosäuren | 0,05 bis 5 % |
| - nichtthioliertes Polypeptid (in Form der aktiven Substanz) | 0,05 bis 8 % |

13. Mittel nach Anspruch 12, dadurch gekennzeichnet, daß die basische Aminosäure in Form von Lysin oder Arginin vorliegt.

14. Mittel nach einem der Ansprüche 12 und 13, dadurch gekennzeichnet, daß das Tocopherol aus 1'-α-Tocopherol, β-Tocopherol, γ-Tocopherol, δ-Tocopherol sowie deren Gemischen ausgewählt ist.

15. Mittel nach einem der Ansprüche 12 und 13, dadurch gekennzeichnet, daß das Tocopherolderivat aus Tocopherolacetat und Tocopherolnikotinat ausgewählt ist.

16. Mittel nach einem der Ansprüche 12 bis 15, dadurch gekennzeichnet, daß es in Form einer für den Schutz vor Oxidation der Hautlipide vorgesehenen Creme vorliegt.
